Europäisches Patentamt

European Patent Office

Office européen des brevets

(19)

(11) Publication number: **0 139 447 B1**

## EUROPEAN PATENT SPECIFICATION

(12)

(45) Date of publication of patent specification: **30.12.92** (51) Int. Cl.⁵: **C12N 9/72**, A61K 37/54

(21) Application number: **84306117.7**

(22) Date of filing: **07.09.84**

The file contains technical information submitted after the application was filed and not included in this specification

(54) **A process for preparing urokinase zymogen.**

(30) Priority: **13.09.83 JP 170354/83**
**17.10.83 JP 195051/83**

(43) Date of publication of application:
**02.05.85 Bulletin 85/18**

(45) Publication of the grant of the patent:
**30.12.92 Bulletin 92/53**

(84) Designated Contracting States:
**BE DE FR GB NL SE**

(56) References cited:
**EP-A- 92 182**
**WO-A-81/01417**
**BE-A- 896 253**

**CHEMICAL ABSTRACTS,vol.97 , 1982, page 406,no.211308x COLUMBUS, OHIO. (US) L.NIELSEN et al.:"Purification of zymogen to plasminogen activator from human glioblastoma cells by affinity chromatography with monoclonal antibody".**

(73) Proprietor: **THE GREEN CROSS CORPORATION**
**15-1, Imabashi-1-chome Higashi-ku Osaka(JP)**

(72) Inventor: **Kasai, Shunji**
**5-6-203, Fujisakanishimachi Hirakata-Shi(JP)**
Inventor: **Arimura, Hirofumi**
**18-1-401, Uenosaka-2-Chome Toyonaka-Shi(JP)**
Inventor: **Mori, Kokage**
**1-5, Sakagami-2-Chome Tarumi-Ku Kobe(JP)**
Inventor: **Nishhida, Masayuki**
**2-6-304, Aoba-3-Chome Shimamotocho Mishima-Gun Osaka(JP)**
Inventor: **Suyama, Tadakazu**
**3-7, Matsuigaoka-4-Chome Tanabecho Tsuzuki-Gun Kyoto(JP)**

(74) Representative: **Vossius & Partner**
**Siebertstrasse 4 P.O. Box 86 07 67 W-8000 München 86(DE)**

PROC. NATL. ACAD.SCI. USA, vol.79, June 1982 pages 3720-3723. K.KALTOFT et al.:"Monoclonal antibody that specifically inhibits a human M 52,000 plasminogen-activating enzyme".

PATENTS ABSTRACTS OF JAPAN,vol.4, no.38 (C-4) (520) March 27,1980 page 109 C 4

CHEMICAL ABSTRACTS,vol.97 , 1982, page 406,no.211308x COLUMBUS, OHIO. (US) L.NIELSEN et al.:"Purification of zymogen to plasminogen activator from human glioblastoma cells by affinity chromatography with monoclonal antibody".

PROC. NATL. ACAD.SCI. USA, vol.79, June 1982 pages 3720-3723. K.KALTOFT et al.:"Monoclonal antibody that specifically inhibits a human M 52,000 plasminogen-activating enzyme".

PATENTS ABSTRACTS OF JAPAN,vol.4, no.38 (C-4) (520) March 27,1980 page 109 C 4

Husain et al (1983), Archives of Biochemistry and biophysics, vol. 220, pp. 31-38

Sueishi et al, Biochimica et Biophisica Acta 717 (1982), pp. 327-338

## Description

This invention relates to a process for preparing an inactive proenzyme of urokinase (urokinase zymogen).

Among the thrombolytic agents heretofore known, the most famous is urokinase. It has been prepared from human urine as well as from culture fluid of human kidney cells, and consists principally of two kinds - one with a molecular weight of 30,000 and the other with a molecular weight of 50,000, as determined by the use of SDS-polyacrylamide gel electrophoresis. The urokinase with higher molecular weight has a higher thrombolytic activity and is more useful as a medicine. Its molecular structure comprises two chains - H-chain (having a molecular weight of 30,000) and L-chain (having a molecular weight of 20,000), linked together with only one disulfide bond. Accordingly, it has a tendency to easily undergo molecular weight diminution by reductive treatment. Thrombolysis by urokinase, however, is liable to systemic plasminogen activation and the resulting degradation of fibrinogen and coagulation proteins in the circulating blood.

On the other hand, another thrombolytic agent which is called tissue plasminogen activator (t-PA) derived from human endothelial cells or melanoma cells is known. It is different in nature from the urokinase in that it has a higher molecular weight of 72,000 containing 527 amino acids, and is shown to be a more specific and effective thrombolytic agent than urokinase because of its high affinity for fibrin and thrombus-localized stimulation of plasminogen activator activity without systemic plasminogen activation and degradation of plasma proteins. However acquisition of sufficient amounts of t-PA has been hampered by its low concentration in cell culture.

WO-A-8101417 discloses the purification of urokinase zymogen from kidney tissue culture medium having a molecular weight of about 56 000, a single chain molecule structure and a selective affinity for fibrin.

Nielsen et al., Biochemistry 1982, 21, 6410-6415, discloses the purification of a zymogen to plasminogen activator from human glioblastoma cells by affinity chromatography with monoclonal antibodies.

EP-A 092 182 (a document according to Article 54(3)(4) EPC) discloses the preparation of a human high molecular weight urokinase via recombinant DNA technology.

Husain et al., Archives of Biochemistry and Biophysics, Vol. 220 (1983), pp 31-38, dicloses the purification of a single chain high molecular weight form of urokinase from human urine.

Sueishi et al., Biochimica et Biophysica Acta 717 (1982) 327-338, discloses the purification of a plasminogen activator from human kidney which is dissimilar to urokinase.

Patent Abstracts of Japan, Volume 4, No. 38, March 27, 1980, page 109 (C-4) discloses the separation of pure urokinase from a crude urokinase preparation by using an adsorbent comprising sawdust or a modified sawdust.

It is the object of the invention to provide a process for preparing a human urokinase zymogen to obtain a superior thrombolytic agent which is easily available. There has been found in serum-free culture fluid of human kidney cells a zymogen of urokinase which has a molecular weight of about 50,000, undergoes no molecular weight diminution by reductive treatment and moreover has a high affinity for fibrin as compared with the known type of urokinase.

Thus, this invention provides a process for preparing a human urokinase zymogen characterized in that urokinase zymogen-producing cells derived from human kidneys are cultivated in a serum-free tissue culture medium supplemented with human albumin, subjecting the supernatant of the culture fluid to a chromatography on a weak cation exchanger at a pH value of 4.5 - 6.5 and eluting the urokinase zymogen-containing fraction with a buffer solution of pH 7.5 - 9.5 and subjecting the eluate to an affinity chromatography using an immobilized antiurokinase zymogen antibody at a pH value of 6 - 8 and eluting the urokinase zymogen with a buffer solution having a pH value of 2 - 4.

The human urokinase zymogen obtained by the process of the invention displays the following features:
(a) molecular weight:
about 50,000 as determined by SDS-polyacrylamide gel electrophoresis under reducing and non-reducing conditions;
(b) primary structure:
single chain polypeptide which is converted after treatment with catalytic amounts of plasmin into the two-chain form of urokinase H and L;
(c) affinity for fibrin:
selective and strong;
(d) fibrinolytic activity:
little when determined with the urokinase substrate S-2444;
(e) specific plasminogen activator (urokinase) activity:

3

little without plasmin treatment and at least 80,000 U/mg protein when converted into urokinase by a treatment with plasmin;

(f) source:

obtainable from human kidney cells; and

(g) glycosylation pattern:

as synthesized in human kidney cells.

Since the present zymogen obtained by the process of the invention has a higher affinity for fibrin than that of hitherto known urokinase, it is useful as a medicine for its clinical effect in thrombolytic therapy.

The human kidney cells to be used in this invention may be obtained, for example, by acquiring a primary culture of diploid cells obtained from human kidney. For example, human kidney is cut into fragments and treated with 0.25 % trypsin. Trypsinized cells are grown in primary culture. The zymogen-producing cells are obtained therefrom by exposing them to a thin layer of fibrin clot and separating cells which exhibit fibrinolytic activity. The cells are inoculated at a rate of 2 - 20 x $10^4$ cells/ml and cultured for about 3 days; when the number of cells has reached about 3 times the number of inoculated cells, a trypsin-EDTA mixed solution is added thereto, and the unilaminar, juvenile cells are collected to be used as the raw material.

The media to be used are for example, Waymouth's medium or Dulbecco's modified MEM medium used for tissue culture. In preliminary culture, 5 % of heat-inactivated bovine fetal serum may be added to the above-mentioned medium. At the stage of culture for producing the present zymogen, cultivation is conducted by the use of a serum-free medium, preferably in admixture with human serum albumin. To the serum-free medium, there may preferably be added human or bovine albumin, lactalbumin hydrolysate, transferrin, amino acids of various kinds, fatty acids of various kinds, and hormones such as insulin. For example, the cells are grown to confluence in serum-supplemented medium. The culture is washed 3 times with serum-free medium and then maintained in serum-free medium supplemented with human serum albumin with intervals of 2 or 3 days between medium changes.

The recovery of the zymogen from the culture fluid can be conducted by concentrating the supernatant of the culture fluid with respect to protein therein and subjecting the protein to affinity chromatography using an antibody column. For example, the concentration may be carried out by suitable combination of such means as centrifugation, vacuum concentration, salt-out fractionation, gel filtration, concentration and ion exchange chromatography.

More particularly, the recovery may be conducted, for example, in the following way. Thus, the culture fluid is first centrifuged to collect the supernatant. The collected supernatant is partly purified by ion exchange chromatography. The most suitable carrier for the chromatography is a weakly acidic cation exchanger, which includes, for example, CM-Sephadex®, CM-Sepharose® (Pharmacia Fine Chemicals) or CM-TOYOPEARL (Toyo Soda Manufacturing Co., Ltd). After conditioning the carrier to pH 4.5 to 6.5, preferably pH 5 to 6, by a buffer solution, the collected solution mentioned above is developed and allowed to be adsorbed on the carrier. The carrier is washed with the same buffer solution as used in the above pH conditioning and then treated with a buffer solution of pH 7.5 to 9.5, more preferably pH 8 to 9, to elute the present zymogen. Examples of the suitable buffer solution include a phosphate buffer solution. The eluted solution is then highly purified by affinity chromatography. The carrier to be used may be either a polyclonal antibody column or a monoclonal antibody column.

In the method using a polyclonal antibody, the anti-zymogen antibody can be obtained by immunizing an animal with the highly purified zymogen, and recovering and purifying the intended antibody from the resulting serum.

The preparation of the above-mentioned anti-serum may be carried out according to known methods. For example, a mixed emulsion prepared from the highly purified zymogen and Freund's complete adjuvant is injected intracutaneously 2 to 3 times to an animal; blood is collected several days after the final immunization; the collected blood is coagulated at room temperature, allowed to stand overnight at 4°C, and then centrifuged at 3,000 rpm (1,500×g) for 20 minutes to give said antiserum.

The animals to be used for immunization are not specifically restricted as to species and include, for example, rats, mice, rabbits, goats and horses. The purification of said antiserum can be conducted, for example, according to the method described in J. Am. Chem. Soc., 62, 3386 (1940) or Fed. Proc., 17, 1161 (1958).

In the method using a monoclonal antibody, the anti-zymogen antibody is obtained by cell fusion. The cell fusion can be conducted by a method known per se. For example, a proliferative cell and a lymphocyte which is producing the intended antibody are made to react in the presence of polyethylene glycol, resulting in formation of a cell which has both proliferative ability and antibody-producing ability. The antibody produced by the cell is a single antibody which reacts only with a single antigenic determinant.

In this invention, though other methods may be adopted, mouse myeloma cells are used as the proliferative cell and splenic cells of mice (B-cells) which have been immunized with the zymogen are used as the antibody-producing lymphocyte to be fused together. Then, those cells which are producing the intended antibody are collected by screening, from which cells the monoclonal antibody to the zymogen is then obtained.

The thus obtained anti-zymogen antibody is immobilized on a matrix to be used in an antibody column.

For immobilizing the anti-zymogen antibody without losing its activity, the following insoluble matrices can be used: copolymer of amino acids [J. Biol. Chem., 236, 1970 (1961)] , cellulose [Nature, 189, 576 (1961)], agarose or Sephadex® [Nature, 215, 1491 (1967); Nature, 245, 3059 (1970)] and polyacrylamide [Biochem., 8, 4074 (1966)]. These means permit effective immobilization of the anti-zymogen antibody according to the well known method of immobilizing an enzyme. By using the adsorbents thus obtained, the zymogen can be obtained in good yield and high purity.

The affinity chromatography of the zymogen according to this invention can be conducted as follows.

The zymogen which has been partly purified by means of a cation exchanger is brought into contact with and adsorbed on an anti-zymogen antibody column which has been equilibrated with a buffer solution of pH 6 to 8. After being washed, the column is eluted with an aqueous solution of pH 2 to 4.

The method of recovery described above gives merely an example of the method of recovering the zymogen of this invention, and it is needless to say that other methods may also be used for the recovery.

Characteristics of the urokinase zymogen obtained according to the process of this invention

(1) Molecular weight

The molecular weight of the zymogen was determined by the use of SDS-polyacrylamide gel electrophoresis [Nature, 227, 680 - 685 (1970)]. The zymogen shows only one single Comassie blue-stainable band with molecular weight of 50,000 on SDS-polyacrylamide gel electrophoresis on 10 % gel under reducing conditions as well as nonreducing conditions, which indicates that the zymogen is a single polypeptide chain of approximately 50,000 molecular weight.

(2) Sensitivity to enzyme

The sensitivity to plasmin was investigated. The result revealed that although the zymogen itself shows little plasminogen activator activity, it comes to manifest significantly the activity by catalytic amounts of plasmin and the extent of the induced activity depends on the concentration (Table 1) and the treating-time (Table 2) in the plasmin treatment. The activity was determined in the following ways.

In the experiments shown in Table 1, a solution of the zymogen was prepared in a concentration of 1 $\mu$g/ml in terms of the zymogen protein. The solution was pretreated for 60 minutes with plasmin of respective concentrations, and the induced enzymatic activities were then determined by colorimetric method with urokinase substrate S-2444 [Claeson et al. Haemostasis, 7, 76 - 78 (1978)].

In the experiments shown in Table 2, a solution was prepared which contained 0.1 $\mu$g/ml of plasmin and 1 $\mu$g/ml of the present zymogen in terms of the zymogen protein, and the effect of plasmin treatment time was determined with the elapse of time.

Similarly, the zymogen which had been pretreated with catalytic amounts of plasmin converted glu-plasminogen, which is a natural substrate for plasminogen activator, into plasmin. However, the zymogen itself could not induce the conversion. Furthermore, the potential activity of the zymogen was completely inhibited by anti-urokinase antibody but not by anti-t-PA antibody.

The zymogen with a single polypeptide chain was cleaved into the two-chain form composed of characteristic urokinase H (molecular weight of 30,000) and L (molecular weight of 20,000) chains when treated with catalytic amounts of plasmin.

These results indicate that the zymogen is an inactive proenzyme of human urokinase and it is converted to two-chain form (urokinase) by catalytic amounts of plasmin. Hereafter, the activity of the zymogen was determined by a colorimetric method with S-2444 after activation to urokinase with plasmin (0.4 $\mu$g/ml) for 1 hr at 37ºC in 0.1 M Tris-HCl, pH 8.1 containing 0.5 % Triton® X-100.

Table 1

| Plasminogen activator activity (U/ml) induced by plasmin | | | | | |
|---|---|---|---|---|---|
| | Without plasmin treatment | Plasmin pretreatment concentration (μg/ml) | | | |
| | | 0.025 | 0.1 | 0.4 | 1.6 |
| Control (without enzyme addition) | 0 | 0 | 0 | 0 | 3 |
| Urokinase | 44 | 52 | 51 | 53 | 57 |
| Zymogen of this invention | 2.5 | 52 | 56 | 80 | 80 |

Table 2

| Time course of increase in plasminogen activator activity of the zymogen induced by plasmin | |
|---|---|
| Plasmin pretreatment time (min.) | Plasminogen activator activity (U/ml) |
| 0 | 2.5 |
| 1 | 4 |
| 2 | 7 |
| 5 | 12 |
| 10 | 26 |
| 20 | 35 |
| 40 | 45 |
| 60 | 56 |

(3) Amino acid composition and sequence

Recently we reported a cDNA sequence coding for human urokinase (Japanese Patent Application No. 37119/84). The deduced amino acid sequence is shown below. The amino acid composition of the zymogen was determined by the use of an automatic amino acid analyzer Model LC-4A (manufactured by Shimadzu Corp.). The amino acid composition of the present zymogen was found to be very similar to that deduced from human urokinase cDNA (Table 3). Furthermore, the $NH_2$-terminal amino acid sequence (residue 1-16) of the zymogen was determined by the use of Gas-phase Protein Sequencer Model 470A (manufactured by Applied Biosystems Inc.) and also found to be identical with that deduced from human urokinase cDNA.

These findings indicate that the present zymogen consisting of 411 amino acids is the precursor form of human urokinase predicted from human urokinase cDNA.

6

```
        1                                      11
Ser-Asn-Glu-Leu-His-Gln-Val-Pro-Ser-Asn-Cys-Asp-Cys-Leu Asn Gly-Gly-Thr-Cys-Val-
       21                                      31
Ser-Asn-Lys-Tyr-Phe-Ser-Asn-Ile-His-Trp-Cys-Asn-Cys-Pro-Lys-Lys-Phe-Gly-Gly-Gln-
       41                                      51
His-Cys-Glu-Ile-Asp-Lys-Ser-Lys-Thr-Cys-Tyr-Glu-Gly-Asn-Gly-His-Phe-Tyr-Arg-Gly-
       61                                      71
Lys-Ala-Ser-Thr-Asp-Thr-Met-Gly-Arg-Pro-Cys-Leu-Pro-Trp-Asn-Ser-Ala-Thr-Val-Leu-
       81                                      91
Gln-Gln-Thr-Tyr-His-Ala-His-Arg-Ser-Asp-Ala-Leu-Gln-Leu-Gly-Leu-Gly-Lys-His-Asn-
      101                                     111
Tyr-Cys-Arg-Asn-Pro-Asp-Asn-Arg-Arg-Arg-Pro-Trp-Cys-Tyr-Val-Gln-Val-Gly-Leu-Lys-
      121                                     131
Pro-Leu-Val-Gln-Glu-Cys-Met-Val-His-Asp-Cys-Ala-Asp-Gly-Lys-Lys-Pro-Ser-Ser-Pro-
      141                                     151
Pro-Glu-Glu-Leu-Lys-Phe-Gln-Cys-Gly-Gln-Lys-Thr-Leu-Arg-Pro-Arg-Phe-Lys-Ile-Ile-
      161                                     171
Gly-Gly-Glu-Phe-Thr-Thr-Ile-Glu-Asn-Gln-Pro-Trp-Phe-Ala-Ala-Ile-Tyr-Arg-Arg-His-
      181                                     191
Arg-Gly-Gly-Ser-Val-Thr-Tyr-Val-Cys-Gly-Gly-Ser-Leu-Ile-Ser-Pro-Cys-Trp-Val-Ile-
      201                                     211
Ser-Ala-Thr-His-Cys-Phe-Ile-Asp-Tyr-Pro-Lys-Lys-Glu-Asp-Tyr-Ile-Val-Tyr-Leu-Gly-
      221                                     231
Arg-Ser-Arg-Leu-Asn-Ser-Asn-Thr-Gln-Gly-Glu-Met-Lys-Phe-Glu-Val-Glu-Asn-Leu-Ile-
      241                                     251
Leu-His-Lys-Asp-Tyr-Ser-Ala-Asp-Thr-Leu-Ala-His-His-Asn-Asp-Ile-Ala-Leu-Leu-Lys-
      261                                     271
Ile-Arg-Ser-Lys-Glu-Gly-Arg-Cys-Ala-Gln-Pro-Ser-Arg-Thr-Ile-Gln-Thr-Ile-Cys-Leu-
      281                                     291
Pro-Ser-Met-Tyr-Asn-Asp-Pro-Gln-Phe-Gly-Thr-Ser-Cys-Glu-Ile-Thr-Gly-Phe-Gly-Lys-
      301                                     311
Glu-Asn-Ser-Thr-Asp-Tyr-Leu-Tyr-Pro-Glu-Gln-Leu-Lys-Met-Thr-Val-Val-Lys-Leu-Ile-
      321                                     331
Ser-His-Arg-Glu-Cys-Gln-Gln-Pro-His-Tyr-Tyr-Gly-Ser-Glu-Val-Thr-Thr-Lys-Met-Leu-
      341                                     351
Cys-Ala-Ala-Asp-Pro-Gln-Trp-Lys-Thr-Asp-Ser-Cys-Gln-Gly-Asp-Ser-Gly-Gly-Pro-Leu-
      361                                     371
Val-Cys-Ser-Leu-Gln-Gly-Arg-Met-Thr-Leu-Thr-Gly-Ile-Val-Ser-Trp-Gly-Arg-Gly-Cys-
      381                                     391
Ala-Leu-Lys-Asp-Lys-Pro-Gly-Val-Tyr-Thr-Arg-Val-Ser-His-Phe-Leu-Pro-Trp-Ile-Arg-
      401                                     411
Ser-His-Thr-Lys-Glu-Glu-Asn-Gly-Leu-Ala-Leu
```

The arrow indicates the potential cleavage site between lysine and isoleucine which generates the two-chain form from the zymogen. The solid bars indicate the potential disulfide bridges based on human urinary urokinase.

7

EP 0 139 447 B1

Table 3

| Comparison of amino acid composition of the zomogen with that deduced from human urokinase cDNA | | |
|---|---|---|
| | The zymogen | Amino acid composition deduced from human urokinase cDNA[a] |
| Aspartic acid | 9.6 | 9.2 |
| Threonine | 6.7 | 6.5 |
| Serine | 7.5 | 7.4 |
| Glutamic acid | 10.4 | 9.9 |
| Proline | 5.9 | 5.7 |
| Glycine | 8.9 | 8.7 |
| Alanine | 4.2 | 4.0 |
| Half-cystine | 4.2 | 6.0 |
| Valine | 4.2 | 4.7 |
| Methionine | 1.1 | 1.7 |
| Isoleucine | 3.9 | 4.7 |
| Leucine | 7.8 | 7.7 |
| Tyrosine | 5.2 | 4.5 |
| Phenylalanine | 3.6 | 3.0 |
| Histidine | 4.1 | 4.2 |
| Lysine | 7.1 | 6.7 |
| Arginine | 5.7 | 5.5 |
| Tryptophan | ND[b] | OM[c] |

[a]Amino acid composition is deduced from human urokinase cDNA.
[b]ND, not determined.
[c]OM, Omitted.

(4) Other properties

Active center: Diisopropyl fluorophosphate (DFP) is a specific reagent for serine residues and reacts with the active site serine residue in urokinase forming a stable modified enzyme. Urokinase incorporated 34-fold more [³H]DFP than the present zymogen. This value is almost consistent with that of stimulation of the plasminogen activator activity. It seems that the cleavage of a single polypeptide chain of the zymogen by plasmin makes the unreactive serine residues of the molecule high reactive.

Urokinase can bind to p-aminobenzamidine via its side-chain binding pocket present in the active center. The zymogen or urokinase dissolved in 0.4 M NaCl, 0.1 M sodium phosphate buffer, pH 7.0 was applied to the p-aminobenzamidine CH-Sepharose®. Only 2 % of the original activity of urokinase passed the column and the adsorbed activity was eluted by changing the buffer to 0.4M NaCl, 0.1 M sodium acetate buffer, pH 4.0. The recovery of activity from the column was 80 % of the original activity. On the other hand, 98 % of the original activity of the zymogen passed the column and only 0.1 % of the original activity was eluted.

These findings indicate that the p-aminobenzamidine-binding pocket as well as the active site present in the active center are not functional in the zymogen.

Secondary structure: When the zymogen was examined for its $\alpha$-helix and $\beta$-content by means of circular dichroism, it showed higher $\alpha$-helix and $\beta$-content than those of previously known urokinase of human urine origin. This indicates that the present zymogen is different in the secondary structure from the prior urokinase.

Affinity for fibrin: The zymogen (5 U as enzyme) was added to a reaction mixture containing 2 mg/ml of plasminogen-free fibrinogen. After coagulation with thrombin, the mixture was incubated at 37°C for 15 minutes. The clot and supernatant were separated by centrifugation and the plasminogen activator activity in the supernatant was determined. The obtained value was regarded as the non-adsorbed amount, and the amount adsorbed to fibrin was calculated as the remainder obtained by substracting the value from the initial amount.

The result is shown in Table 4 together with that of urokinase.

The zymogen obtained according to the process of this invention has a selective strong affinity for

8

fibrin, and is similar in this respect to tissue plasminogen activator (t-PA). This is of great importance in thrombolytic therapy. Thus, with the usual urokinase, the rapid inactivation of plasmin necessitates a large dose of urokinase, which is liable to cause a serious side effect such as hemorrhagic tendency. On the other hand, the zymogen obtained according to the process of this invention has a high affinity for fibrin and hence permits the fibrinolysis to take place confinedly on the solid phase (fibrin). Thus, it provides an ideal medicine for thrombolytic therapy.

Table 4

| | Proportion adsorbed to fibrin clot |
|---|---|
| Zymogen of this invention | 66 % |
| Urokinase | 10 % |

The thrombolytic ability: The solubilizing ability for a fibrin thrombus formed from human plasma was examined. A fibrin thrombus labelled with $^{125}$I was incubated at 37°C for 3 hours in human plasma containing the zymogen of this invention or a known human urinary urokinase, and the radioactivity of dissolved fibrin was determined. The results are shown in Table 5. The results have revealed that the thrombolytic ability of the zymogen of this invention is about three times as high as that of known human urinary urokinase.

Table 5

| | Dissolved proportion |
|---|---|
| Zymogen obtained according to the process of of this invention | |
| 500 U/ml | 97 % |
| Urokinase | |
| 500 IU/ml | 40 % |
| 1,000 IU/ml | 68 % |
| 1,500 IU/ml | 84 % |

Fibrinogenolytic activity: The known human urinary urokinase decomposes, besides the fibrin at the thrombus site, also fibrinogen and coagulation factors(factor V, factor VIII and factor XIII) present in plasma, and so causes the side effect of increasing the hemorrhagic tendency. Accordingly, the degradation of fibrinogen in plasma by the zymogen of this invention was investigated.

To a human plasma, to which a fibrinogen labelled with $^{125}$I had been added beforehand, was added the zymogen obtained according to the process of this invention (500 U/ml) or human urinary urokinase (1,500 IU/ml), and the mixture was incubated at 37°C. Samples of the mixture were withdrawn after 2, 10, 60, 120 and 180 minutes of incubation and examined by SDS-polyacrylamide gel electrophoresis and autoradiography to determine the extent of degradation of $^{125}$I-labelled fibrinogen with time.

The fibrinogen having a molecular weight of 330,000 was degraded by plasmin into fragments of molecular weights of 240,000, 155,000, 85,000 and 50,000 and other small fragments.

When the human urinary urokinase (1,500 IU/ml) was incubated in plasma conaining $^{125}$I-labeled fibrinogen, significant degradation of fibrinogen was observed in 10 minutes and the degradation proceeded further with time.

In contrast, no fibrinogen degradation was observed in the incubation with the zymogen at 500 U/ml, where complete plasma clot lysis would be expected.

Thus, it can be inferred that, since the zymogen of this invention has a high affinity for fibrin and a high fibrinolytic ability and yet does not decompose the fibrinogen present in plasma, it will decompose only that fibrin which is the main component of a thrombus and will cause little risk of increasing the hemorrhagic tendency attendant upon the decrease of fibrinogen in blood which is often encountered as a side effect of a large dose of urokinase.

Stability in plasma: The molecular weight and the single-chain structure of the zymogen of this invention in plasma were examined to investigate its stability. The zymogen obtained according to the process of this invention labelled with $^{125}$I 500 (U/ml) was allowed to stand in human plasma at 37ºC.Samples were withdrawn after 1, 2 and 3 hours of standing and each divided into two portions. One portion was modified with 1 % SDS and the other portion was subjected to reductive treatment with 1 % SDS and 1 % 2-mercaptoethanol. The treated samples were examined by SDS-polyacrylamide gel electrophoresis and autoradiography. The result revealed that even after 3 hours of standing both the specimen subjected to no reduction and the one subjected to reductive treatment gave the same migration pattern as that at zero hour of standing, exhibiting a single band corresponding to a molecular weight of about 50,000. Accordingly, it may be concluded that the molecular weight and the single-chain structure of the zymogen obtained according to the process of this invention are stable in plasma.

Furthermore, the urokinase activity of the zymogen obtained according to the process of this invention in plasma was examined by the aforementioned synthetic substrate method without plasmin treatment. No activity was observed.

From the foregoing, it may be concluded that the zymogen of this invention is stable as a zymogen in plasma.

Mechanism of thrombolysis: From the properties of the zymogen obtained according to the process of this invention described above, it is inferred that the present zymogen differs in the mechanism of thrombolysis from known human urinary urokinase.

The human urinary urokinase acts directly on plasminogen present in plasma as well as on plasminogen adsorbed to a thrombus, and the plasmin thus formed decomposes fibrinogen and fibrin.

In the case of the zymogen obtained according to the process of this invention, on the other hand, it appears that, since it exhibits no plasminogen activator activity in plasma and has a high affinity for fibrin, it can easily arrive at the thrombus site, is combined there with fibrin, is converted to urokinase on the thrombus by the action of a trace amount of plasmin contained in the thrombus. It appears further that the formed urokinase then converts the plasminogen bonded to the fibrin molecule into plasmin and thus decomposes the fibrin.

Thus, by the use of the zymogen obtained according to the process of this invention, it is possible to effect a fibrinolysis confined only on the solid phase, fibrin (thrombus). Therefore, it has a bright prospect as a new type of thrombolytic agent.

The zymogen obtained according to the process of the present invention can also be used as a reagent for chemical, pharmacological or medical use. When used as a medicine, it may, as desired, be subjected to heat-treatment, aseptically filteration, lyophilization, dispensation into vials, or made up into various medical preparations according to customary methods for producing medicines. However, the present zymogen has a tendency to be adsorbed on a glass wall, and has a poor stability against heat. Also, since it has a poor storage stability in solution, it would be thought desirable to have a lyophilized product of it for use as a medicine. Nevertheless, the zymogen has actually been found to lose its activity even during the lyophilization. Accordingly, it is necessary to stabilize the zymogen by some means.

The present inventors made an extensive study to find a stabilizer of the zymogen and selected albumin or a nonionic surface active agent such as Triton® X-100 or Tween® 80. In the course of or after the purification step, they are added to the aqueous solution of the zymogen.

Among them albumin is most desirable, and when it is made to exist together with the zymogen, the zymogen is stabilized in an aqueous solution, is not inactivated during lyophilization, and shows an improved storage stability as a lyophilized preparation.

The albumin used is preferably of human origin in view of the problem of antigenicity. There is no further restriction as to the property of albumin so long as it has been purified to suit medical use. The purity is preferably such that the albumin content is at least 80 % as determined by electrophoresis. As the method for preparing a suitable human albumin, there may be mentioned, for example, the ethanol fractionation method (Japanese Patent Publication Nos. 2869/72 and 5297/60) and a method which comprises heating the albumin fraction in the presence of an organic acid (Japanese Patent Publication Nos. 1604/68 and 401321/76). Particularly preferably used is the albumin which has been heat-treated (preferably at about 60ºC for about 10 hours) to inactivate hepatitis virus and the like.

The compounding ratio of albumin and the present zymogen should correspond to at least 30 mg of albumin relative to 10,000 to 1,000,000 U of the zymogen and preferably corresponds to 30 to 50 mg of albumin relative to 10,000 to 1,000,000 U of the zymogen.

In the lyophilization of an aqueous solution containing the present zymogen, addition of albumin to the aqueous solution to a concentration of at least 3 mg/ml, preferably at least 5 mg/ml, is sufficient for the stabilization of the present zymogen irrespective of the zymogen content in the solution. It is needless to say that, in addition to albumin, other stabilizers may also be added to the composition. For example, inorganic or organic salts are favorably added to the composition.

Lyophilization of the zymogen in the presence of albumin may be carried out, for example, as follows. An aqueous solution containing the present purified zymogen is adjusted to pH 5 to 9; albumin is added thereto in a sufficient amount for stabilization mentioned above; the resulting aqueous solution is aseptically filtered, dispensed into vials and then lyophilized by a customary method.

The composition thus obtained undergoes no loss of zymogen especially during the preparation step, shows an excellent stability during storage, and can favorably be used as a medicine.

This invention will be illustrated in more detail below with reference to Examples, but it is in no way limited thereto.

Example 1

Human kidney cells were cultured for 3 days in a serum-free medium (Waymouth's medium) to which 0.1 % human serum albumin had been added. The resulting culture fluid was centrifuged and the supernatant was frozen and stored. The pooled supernatant of the culture fluid was adjusted to pH 5.5 and then brought into contact with CM-Sephadex® C-50. The column was washed with 0.16 M phosphate buffer solution, pH 5.5, and the zymogen which had been adsorbed on the column was then eluted with 0.16 M phosphate buffer solution, pH 8.5.

In the meantime, splenic cells of mouse BALB/c which had been immunized beforehand with the zymogen and mouse myeloma cells were fused together in the presence of polyethylene glycol. From the resulting hybridoma, those clones which showed a high producing capacity of antibody for the zymogen were selected. The anti-zymogen monoclonal antibody was recovered from the culture fluid of the fused cell. The monoclonal antibody was immobilized on CNBr-activated Sepharose® 4B (Pharmacia Inc.).

The monoclonal antibody column thus prepared was equilibrated with 0.1 M phosphate buffer solution, pH 7.0, containing 0.4 M NaCl, and the above-mentioned eluate containing the zymogen was brought into contact with the column. The column was washed with 0.1 M phosphate buffer solution, pH 7.0, containing 0.4 M NaCl, and then the zymogen which had been adsorbed on the column was eluted with 0.2 M aqueous glycine-HCl solution, pH 2.5, containing 0.5 M NaCl. The eluate was aseptically filtered and then lyophilized to give a highly purified zymogen having a specific activity of at least 80,000 U/mg.

In SDS-polyacrylamide gel electrophoresis, the purified product showed a single band corresponding to a molecular weight of 50,000 under reducing condition as well as nonreducing condition.

Example 2

To a solution containing 24,000 U/ml of the present zymogen having a specific activity of at least 80,000 U/mg which had been prepared according to the method of Example 1, adjusted to pH 7 with a phosphate buffer solution, was added human serum albumin to a concentration of 35 mg/ml.

The resulting solution was aseptically filtered, dispensed in 2 ml portions into vials of 10 ml volume, and lyophilized at temperatures finally reaching 25°C.

The moisture content of the lyophilized product determined according to "general method, criterion for biological preparations" was found to be about 0.2 %. All of the lyophilized products dissolved immediately on addition of 2 ml of distilled water for injection, giving clear, colorless solutions. The remaining proportions of the zymogen determined on these solutions all showed that the amount of the zymogen had not changed substantially from that before lyophilization. Further, in the preparation step, no loss of the zymogen due to its adsorption to the glass wall was observed.

Example 3

To confirm the stabilization effect, the following experiment was carried out.

To a solution containing $1 \times 10^4$ U/ml, $5 \times 10^4$ U/ml or $5 \times 10^5$ U/ml of the present zymogen which had been purified as described in Example 1, was added human serum albumin to various concentrations ranging from 1 to 100 mg/ml, and the mixtures were then lyophilized. The titer of the lyophilized product was determined immediately after lyophilization and after storage of 3 months at 50°C. The proportions of remaining activity relative to the titer immediately after addition of human serum albumin were as shown in Table 6.

Table 6

| Zymogen Concentration (U/ml) | Albumin Concentration (mg/ml) | Remaining activity (%) | |
|---|---|---|---|
| | | immediately after liophilization | 3 month's storage |
| $1 \times 10^4$ | 0 | 50 | 44 |
| $1 \times 10^4$ | 10 | 55 | 50 |
| $1 \times 10^4$ | 30 | 63 | 62 |
| $1 \times 10^4$ | 50 | 69 | 68 |
| $1 \times 10^4$ | 100 | 68 | 68 |
| $5 \times 10^4$ | 30 | 66 | 65 |
| $1 \times 10^5$ | 30 | 65 | 64 |

## Claims

1. A process for preparing a human urokinase zymogen displaying the following features:
   (a) molecular weight:
   about 50,000 as determined by SDS-polyacrylamide gel electrophoresis under reducing and non-reducing conditions;
   (b) primary structure:
   single chain polypeptide which is converted after treatment with catalytic amounts of plasmin into the two-chain form of urokinase H and L;
   (c) affinity for fibrin:
   selective and strong;
   (d) fibrinolytic activity:
   little when determined with the urokinase substrate S-2444;
   (e) specific plasminogen activator (urokinase) activity:
   little without plasmin treatment and at least 80,000 U/mg protein when converted into urokinase by a treatment with plasmin;
   (f) source:
   obtainable from human kidney cells; and
   (g) glycosylation pattern:
   as synthesized in human kidney cells;
   characterized in that urokinase zymogen-producing cells derived from human kidneys are cultivated in a serum-free tissue culture medium supplemented with human albumin, subjecting the supernatant of the culture fluid to a chromatography on a weak cation exchanger at a pH value of 4.5 - 6.5 and eluting the urokinase zymogen-containing fraction with a buffer solution of pH 7.5 - 9.5 and subjecting the eluate to an affinity chromatography using an immobilized antiurokinase zymogen antibody at a pH value of 6 - 8 and eluting the urokinase zymogen with a buffer solution having a pH value of 2 - 4.

2. The process according to claim 1, wherein the tissue-culture medium used is Waymouth's medium or Dulbecco's modified MEM medium.

3. The process according to claim 1 or 2, wherein a serum albumin, a lactalbumin hydrolysate, a transferrin, an amino acid, a fatty acid, or insulin is added to the culture medium.

4. The process according to any one of claims 1 to 3, wherein the antiurokinase zymogen antibody used is a polyclonal antibody.

5. The process according to any one of claims 1 to 3, wherein the antiurokinase zymogen antibody used is a monoclonal antibody.

## Patentansprüche

1. Verfahren zur Herstellung eines menschlichen Urokinase-Zymogens mit den folgenden Eigenschaften:
   (a) Molekulargewicht:
   etwa 50 000, bestimmt durch SDS-Polyacrylamidgelelektrophorese unter reduzierenden und nicht-reduzierenden Bedingungen;
   (b) Primärstruktur:
   Einketten-Polypeptid, das nach Behandlung mit katalytischen Mengen von Plasmin in die Zweikettenform von Urokinase H und L umgewandelt wird,
   (c) Affinität für Fibrin:
   selektiv und hoch,
   (d) fibrinolytische Aktivität:
   gering, wenn sie mit dem Urokinasesubstrat S-2444 bestimmt wird;
   (e) spezifische Plasminogen-Aktivator-(Urokinase)-Aktivität:
   gering, ohne Plasminbehandlung und mindestens 80 000 E/mg Protein, wenn es durch Behandlung mit Plasmin in Urokinase umgewandelt wird;
   (f) Quelle:
   erhältlich aus menschlichen Nierenzellen; und
   (g) Glykosylierungsmuster:
   wie bei der Synthese in menschlichen Nierenzellen,
   **dadurch gekennzeichnet**, daß die Urokinase-Zymogen produzierenden Zellen, die von menschlichen Nieren stammen, in einem serumfreien Gewebskulturmedium gezüchtet werden, das mit menschlichem Albumin ergänzt ist, der Überstand der Kulturflüssigkeit einer Chromatographie an einem schwachen Kationenaustauscher bei einem pH-Wert von 4,5 bis 6,5 unterworfen wird und die Urokinase-Zymogen enthaltende Fraktion mit einer Pufferlösung von pH 7,5 bis 9,5 eluiert wird und das Eluat einer Affinitätschromatographie unter Verwendung eines immobilisierten anti-Urokinase-Zymogen-Antikörpers bei einem pH-Wert von 6 bis 8 unterworfen wird und das Urokinase-Zymogen mit einer Pufferlösung mit einem pH-Wert von 2 bis 4 eluiert wird.

2. Verfahren nach Anspruch 1, wobei das eingesetzte Gewebskulturmedium Waymouth's-Medium oder Dulbecco's modifiziertes MEM-Medium ist.

3. Verfahren nach Anspruch 1 oder 2, wobei Serumalbumin, Lactalbuminhydrolysat, Transferrin, eine Aminosäure, eine Fettsäure oder Insulin dem Kulturmedium zugesetzt wird.

4. Verfahren nach einem der Ansprüche 1 bis 3, wobei der eingesetzte anti-Urokinase-Zymogen-Antikörper ein polyclonaler Antikörper ist.

5. Verfahren nach einem der Ansprüche 1 bis 3, wobei der eingesetzte anti-Urokinase-Zymogen-Antikörper ein monoclonaler Antikörper ist.

**Revendications**

1. Procédé de préparation d'un zymogène d'urokinase humain présentant les caractéristiques suivants :
   (a) poids moléculaire :
   environ 50.000, déterminé par électrophorèse sur gel SDS-polyacrylamide dans des conditions réductrices et non réductrices ;
   (b) structure primaire :
   polypeptide à chaîne unique qui est converti après traitement par des quantités catalytiques de plasmine en forme d'urokinase H et L à deux chaînes ;
   (c) affinité pour la fibrine :
   sélective et forte ;
   (d) activité fibrinolytique :
   faible par détermination avec le substrat d'urokinase S-2444 ;
   (e) activité d'activateur plasminogène spécifique (urokinase) :
   faible sans traitement avec de la plasmine et d'au moins 80.000 U/mg protéine après conversion en urokinase par un traitement avec de la plasmine ;
   (f) source :
   provient de cellules rénales humaines ; et

(g) schéma de glycosylation :

identique à la synthèse telle qu'elle se déroule dans les cellules rénales humaines ;

caractérisé en ce que des cellules productrices de zymogène d'urokinase provenant de reins humains sont cultivées dans un milieu de culture de tissu sans sérum complété par de l'albumine humaine, en soumettant le surnageant du liquide de culture à une chromatographie sur échangeur de cations faible à un pH de 4,5 à 6,5 et en éluant la fraction contenant le zymogène d'urokinase avec une solution tampon d'un pH de 7,5 à 9,5 et en soumettant l'éluat à une chromatographie d'affinité à l'aide d'un anticorps de zymogène antiurokinase immobilisé, à un pH de 6 à 8 et en éluant le zymogène d'urokinase avec une solution tampon ayant un pH de 2 à 4.

2. Procédé selon la revendication 1, dans lequel le milieu de culture de tissu utilisé est un milieu de Waymouth ou un milieu MEM modifié de Dulbecco.

3. Procédé selon la revendication 1 ou 2, dans lequel une sérumalbumine, un hydrolysat de lactalbumine, une transferrine, un acide aminé, un acide gras, ou de l'insuline est ajouté dans le milieu de culture.

4. Procédé selon l'une quelconque des revendications 1 à 3, dans lequel l'anticorps de zymogène antiurokinase utilisé est un anticorps polyclonal.

5. Procédé selon l'une quelconque des revendications 1 à 3, dans lequel l'anticorps de zymogène antiurokinase utilisé est un anticorps monoclonal.